# EUROPEAN PATENT SPECIFICATION

(11) **EP 2 451 397 B1**
(45) Date of publication and mention of the grant of the patent: **23.09.2015**
(21) Application number: 10797409.9
(22) Date of filing: 12.07.2010
(51) Int. Cl.: A61F 2/32, A61B 17/16, A61F 2/34, A61F 2/36, A61F 2/30

(54) **HIP JOINT DEVICE**
HÜFTGELENKVORRICHTUNG
DISPOSITIF D'ARTICULATION DE LA HANCHE

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, CH-6300 Zug (CH)
(86) International application number: PCT/SE2010/050821
(87) International publication number: WO 2011/005202

(56) References cited:
- EP-A2- 0 945 109
- EP-B1- 0 208 578
- WO-A1-03/049649
- FR-A- 1 047 640
- US-A- 4 024 588
- US-A- 4 044 403
- US-A- 4 159 544
- US-A1- 2003 236 572
- US-A1- 2005 085 915
- US-B1- 6 290 727
- US-B2- 6 986 792

## Description

### TECHNICAL FIELD

The invention relates generally to hip joint prosthesis.

### BACKGROUND

Hip joint Osteoarthritis is a syndrome in which low-grade inflammation results in pain in the hip joints, caused by abnormal wearing of the Cartilage that acts as a cushion inside if the hip joint. This abnormal wearing of the cartilage also results in a decrease of the joints lubricating fluid called Synovial fluid. Hip joint Osteoarthritis is estimated to affect 80% of all people over 65 years of age, in more or less serious forms.

The present treatment for hip osteoarthritis comprises NSAID drugs, local injections of Hyaluronic acid or Glucocorticoid to help lubricating the hip joint, and replacing parts of the hip joint with a prosthesis through hip joint surgery.

The replacing of parts of the hip joint is one of the most common surgeries to date performed at hundreds of thousands of patients in the world every year. The most common method comprises placing a metal prosthesis in Femur and a plastic bowl in Acetabulum. This operation is done through an incision in the hip and upper thigh and through Fascia Lata and the lateral muscles of the thigh. To get access to the joint, the supporting Fibrous Capsule attached to Femur and Ilium needs to be penetrated, making it difficult to get a fully functional joint after the surgery. Femur is then cut at the neck with a bone saw and the prosthesis is placed in femur either with bone cement or without. Acetabulum is slightly enlarged using an Acetabular reamer, and the plastic bowl is positioned using screws or bone cement.

The complications after hip joint surgery includes dislocation of the hip joint and loosening of the prosthesis from its fixation in the femoral bone. The loosening and/or dislocation of the prosthesis could be induced by an abnormal strain being placed on the hip joint from e.g. the patient falling or making a rapid movement of the hip. A completely fixed hip joint prosthesis, without the possibility to dislocate would increase the risk of the prosthesis loosening from its fixation in the femoral bone, since the entire strain is then placed on the femoral bone.

A hip joint prosthesis that could reduce the complications after hip joint surgery would therefore be desirable.

D1 EP0945109 (to Ochoa et al.) discloses a hip joint prosthesis in which a prosthetic caput femur can be inserted into a prosthetic acetabulum by means of a snap-fit connection.

### SUMMARY

A medical device for implantation in a hip joint of a patient is provided. The medical device comprises a first and second piece and a releasing member adapted to, in a first state hold the first piece attached to the second piece, and in a second state release the first piece from the second piece. The releasing member is adapted to change from the first state to the second state when a pre-determined strain is placed on the releasing member.

The medical device comprises, according to the invention, a calibration member for calibrating the pre-determined strain required for said releasing member to change from said first state to said second state. The calibration member could be a calibration screw.

### BRIEF DESCRIPTION OF THE DRAWINGS

The invention is now described, by way of example, with reference to the accompanying drawings, wherein figures 11-15 show the present invention. in which:
Fig. 1 shows the hip joint in section.
Fig. 2 shows the first step in a conventional hip joint surgery,
Fig. 3 shows the step of removing the caput femur from the hip joint capsule,
Fig. 4 shows the incisions made in a laparoscopic/arthroscopic method,
Fig. 5 shows the instruments used in a laparoscopic/arthroscopic method,
Fig. 6 shows the step of creating a hole in the pelvic bone of a patient,
Fig. 7 shows details of a laparoscopic operation,
Fig. 8 shows the patient in section when a laparoscopic operation is performed,
Fig. 9 shows the hip joint in section when a medical device has been provided, in a first state,
Fig. 10 shows the hip joint in section when a medical device has been provided, in a second state,
Fig. 11 shows the hip joint in section when a medical device has been provided, in a first state,
Fig. 12 shows the hip joint in section when a medical device has been provided, in a second state,
Fig. 13 shows the medical device in section,
Fig. 14 shows an alternative embodiment of the medical device shown in fig. 11, in a first state,
Fig. 15 shows an alternative embodiment of the medical device shown in fig. 11, in a second state,
Fig. 16 shows the hip joint in section, when a medical device according to yet another embodiment is provided, in a first state,
Fig. 17 shows the hip joint in section, when a medical device according to yet another embodiment is provided, in a second state,
Fig. 18a shows the hip joint in section when a medical device comprising an elastic or rupture band has been provided, in a first state,
Fig. 18b shows the medical device of fig. 18a, in section, in a first state,
Fig. 19a shows the hip joint in section when a medical device comprising an elastic or rupture band is provided, in a second state,
Fig. 19b shows the medical device of fig. 19a, in section, in a second state,
Fig. 20 shows the hip joint in section, when a medical device according to yet another embodiment has been provided, in a first state,
Fig. 21 shows the hip joint in section, when a medical device according to yet another embodiment has been provided, in a second state,
Fig. 22 shows the hip joint in section, when a medical device comprising a rupture band has been provided, in a first state,
Fig. 23 shows the hip joint in section, when a medical device comprising a rupture band has been provided, in a second state,
Fig. 24 shows the hip joint in section, when a medical device according to yet another embodiment has been provided, in a first state,
Fig. 25 shows the hip joint in section, when a medical device according to yet another embodiment has been provided, in a second state,
Fig. 26 shows the hip joint in section when a medical device, according to an embodiment where the artificial acetabulum surface comprises elastic elements, has been provided, in a first state,
Fig. 27 shows the hip joint in section when a medical device, according to an embodiment where the artificial acetabulum surface comprises elastic elements, has been provided, in a second state,
Fig. 28 shows an alternative embodiment of the medical device shown in fig. 26,
Fig. 29 shows the hip joint in section when a medical device adapted to hold the caput femur 5, or an artificial replacement therefore, to the artificial acetabulum by means of magnetic force, has been provided, in a first state,
Fig. 30 shows the hip joint in section when a medical device adapted to hold the caput femur 5, or an artificial replacement therefore, to the artificial acetabulum by means of magnetic force, has been provided, in a second state,
Fig. 31 shows, schematically, the artificial acetabulum or artificial caput femur,
Fig. 32a shows the artificial acetabulum or artificial caput femur, in section,
Fig. 32b shows an alternative embodiment,
Fig. 33 shows the principle of an alternative embodiment,
Fig. 34 shows the principle of an alternative embodiment,
Fig. 35 shows the principle of an alternative embodiment,

### DETAILED DESCRIPTION

Elasticity is to be understood as a materials ability to deform in an elastic way.

Elastic deformation is when a material deforms under stress (e.g. external forces), but returns to its original shape when the stress is removed. A more elastic material is to be understood as a material having a lower modulus of elasticity. The elastic modulus of an object is defined as the slope of its stress-strain curve in the elastic deformation region. The elastic modulus is calculated as stress / strain, where stress is the force causing the deformation, divided by the area to which the force is applied; and strain is the ratio of the change caused by the stress.

Stiffness is to be understood as the resistance of an elastic body to deformation by an applied force.

Functional hip movements are to be understood as movements of the hip that at least partly correspond to the natural movements of the hip. On some occasions the natural movements of the hip joint might be somewhat limited or altered after hip joint surgery, which makes the functional hip movements of a hip joint with artificial surfaces somewhat different than the functional hip movements of a natural hip joint.

The functional position of an implantable medical device or prosthesis is the position in which the hip joint can perform functional hip movements. The final position is to be understood as a functional position in which the medical device needs no further position change.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

Form fitting is to be understood as an element having a part or section which is adapted to enable a mechanical connection of said element to at least one other element using said part or section. Form fitted structure is a structure of an element which enables form fitting.

The medical device according to any of the embodiments could comprise at least one material selected from a group consisting of: polytetrafluoroethylene (PTFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/or carbon fibers and/or boron, a combination of metal and plastic materials, a combination of metal and carbon based material, a combination of carbon and plastic based material, a combination of flexible and stiff materials, a combination of elastic and less elastic materials, Corian or acrylic polymers.

In the following a detailed description of preferred embodiments of the present invention will be given. In the drawing figures, like reference numerals designate identical or corresponding elements throughout the several figures. It will be appreciated that these figures are for illustration only and are not in any way restricting the scope of the invention. Thus, any references to direction, such as "up" or "down", are only referring to the directions shown in the figures. Also, any dimensions etc. shown in the figures are for illustration purposes.

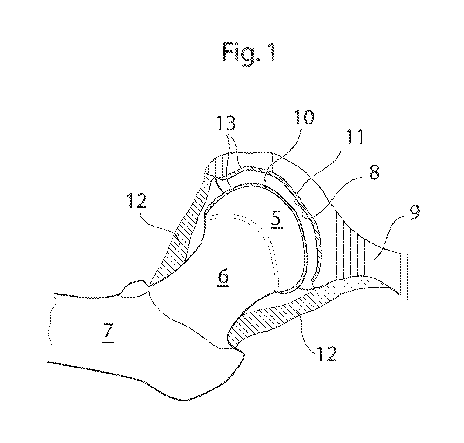

Fig. 1 shows the hip joint of a human patient in section. The hip joint comprises a caput femur 5, or an artificial replacement therefore, placed at the very top of collum femur 6 which is the top part of the femoral bone 7. The caput femur 5, is in connection with the acetabulum 8 which is a bowl shaped part of the pelvic bone 9. Both the caput femur surface 10 and the acetabulum surface 11 is covered with articular cartilage 13 which acts as a cushion in the hip joint. In patients with hip joint osteoarthritis, this articular cartilage 13 is abnormally worn down due to a low grade inflammation. The hip joint is surrounded by the hip joint capsule 12 which provides support for the joint and hinders luxation. After conventional hip joint surgery, penetrating the hip joint capsule 12, the capsule 12 is dramatically weakened due to the limited healing possibilities of its ligament tissue. By performing hip joint surgery without damaging the hip joint capsule 12 the patient can fully recover and place equal amount of strain on an artificial joint as is possible on a natural one.

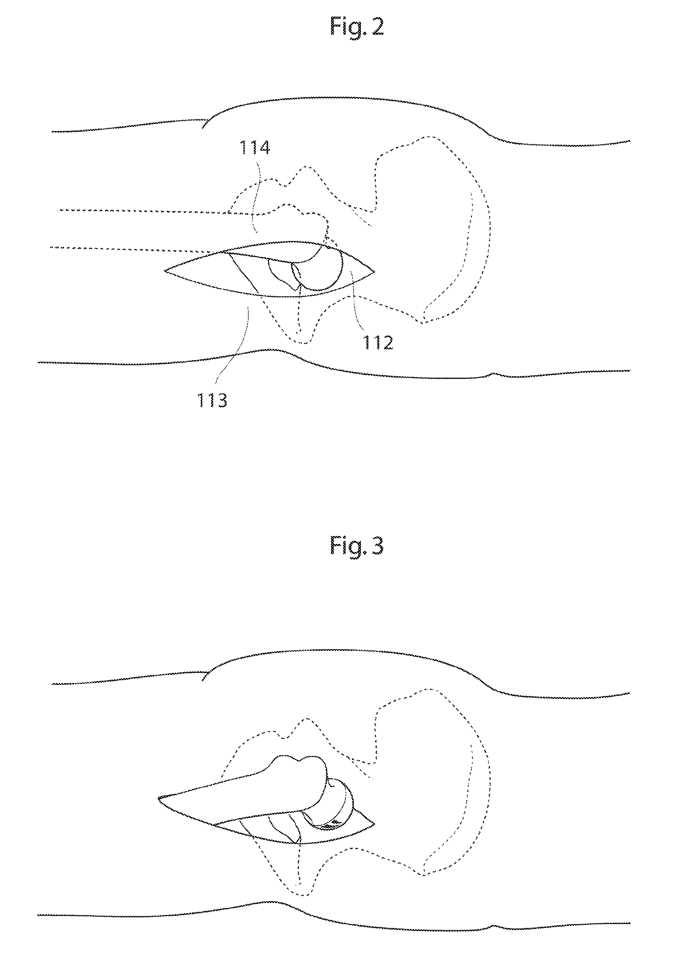

Fig. 2 shows a lateral view of a conventional hip joint surgery where an incision 112 is made in the thigh 113 enabling the surgeon to reach the femoral bone 7 on which the caput femur 5 is located. The femoral bone 7 is then extracted from the hip joint capsule 12 exposing the caput femur 5, which is replaced or resurfaced during the operation.

Fig. 3 shows the placing of an artificial caput femur surface 45 on the caput femur 5 in conventional surgery. However according to other embodiments of the state of the art the entire collum femur 6 is removed using a bone saw, after which a prosthetic part comprising the caput femur is fixated in the femoral bone using bone cement or mechanical fixating members. A bowl shaped cup is then placed in the acetabulum 8 to act as the contacting surface against the new artificial caput femur 45 when the hip joint is performing functional hip movements in its functional position. According to prior art, the artificial caput femur surface and the artificial acetabulum surface is being kept together by means of the hip joint capsule, which is dramatically weakened when the capsule has been penetrated during an operation.

An alternative way of operating a hip joint will now be described.

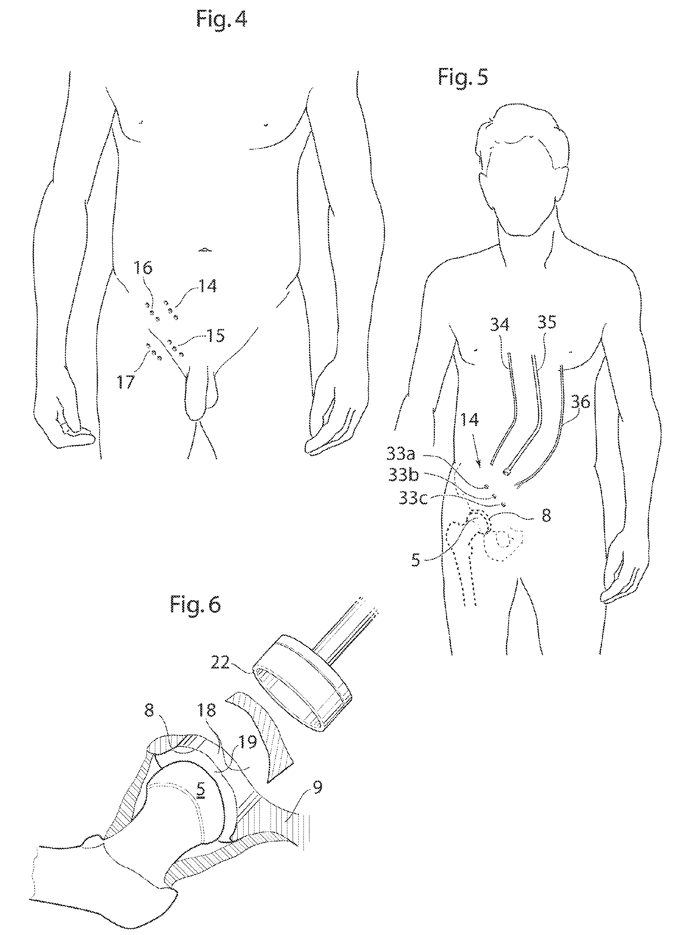

Fig 4 shows a frontal view of the body of a human patient. A laparoscopic/arthroscopic method of operating the hip joint, from the opposite side from acetabulum, is according to a first embodiment performed starting with making small incisions 14 in the abdominal wall of the human patient. The small incisions enable the surgeon to insert laparoscopic trocars into the abdomen of the human patient. According to the first embodiment the incisions 14 passes through the rectus abdominis and peritoneum in to the abdomen of the human patent. According to a second preferred embodiment the small incisions 15 is conducted through the rectus abdominis and in to the pelvic area, below peritoneum. According to a third embodiment the small incisions 16 is performed just between Ilium and the surrounding tissue, an incision 16 which could enable the pelvic bone to be dissected with very little penetration of fascia and muscular tissue. According to a fourth embodiment the incision 17 is made in the inguinal channel. In all of the four embodiments the tissue surrounding the pelvic bone 9 in the area opposite to acetabulum 8 is removed or penetrated which enables the surgeon to reach the pelvic bone 9.

It is obvious that the methods described may both be combined or altered reaching the same goal to dissect the pelvic bone on the opposite side of the acetabulum.

After dissecting the pelvic bone 9 a hole 18 is created in the bone 9, as shown in fig. 6. The hole 18 passes through the pelvic bone from the opposite side from acetabulum 8 and into the hip joint 19.

Fig. 5 shows a frontal view of the body of a human patient, illustrating the laparoscopic method of operating the hip joint from the opposite side from acetabulum 8. The hip joint comprises the acetabulum 8 and the caput femur 5. The small incisions 14 in the abdominal wall of the human patient allows the insertion of laparoscopic trocars 33a,b,c into the body of the patients. Whereafter one or more camera 34, a surgical instrument adapted to create a hole in the pelvic bone 35, or instruments 36 for introducing, placing, connecting, attaching, creating or filling prosthesis or prosthetic parts, can be inserted into said body through said laparoscopic trocars 33a,b,c.

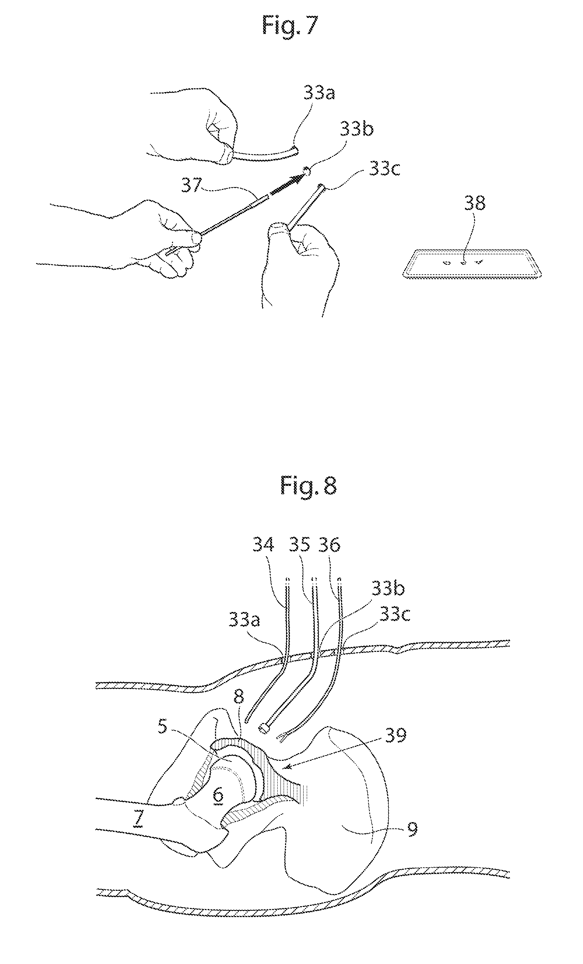

Fig. 7 shows a close-up of the insertion 37 of prosthetic parts 38 into the patient's body through said laparoscopic trocars 33a,b,c. The prosthetic parts could be parts of the artificial caput femur 45, the artificial acetabulum 65 or prosthetic parts or bone material adapted to be used to close the hole 18 created in the pelvic bone 9.

Fig. 8 shows a lateral view of the body of a human patient, with the hip joint shown in section. The hip joint comprises a caput femur 5 placed at the very top of collum femur 6 which is the top part of the femur bone 7. The caput femur 5 is in connection with the acetabulum 8 which is a bowl shaped part of the pelvic bone 9. Laparoscopic trocars 33a,b,c is being used to reach the hip joint 39 with one or more camera 34, a surgical instrument 35 adapted to create a hole in the pelvic bone 9, or instruments 36 for introducing, placing, connecting, attaching, creating or filling prosthesis or prosthetic parts.

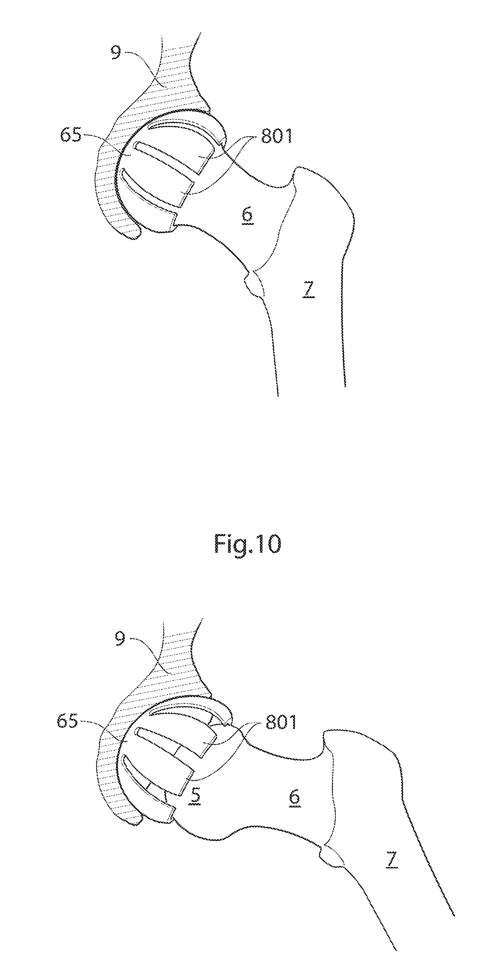

Fig. 9 shows an artificial bowl shaped acetabulum cup 65 placed in the pelvic bone 9. The artificial bowl shaped acetabulum cup 65 comprises releasing members 801 adapted, in a first state, to hold the caput femur 5 which is a ball shaped piece attached to the collum femur 6 in position in the hip joint to the artificial bowl shaped acetabulum cup 65 placed in the pelvic bone 9. In a second state the releasing member 801 is adapted to release the caput femur 5, or an artificial replacement therefore, from the artificial bowl shaped acetabulum cup 65 placed in the pelvic bone 9. The releasing member 801 is adapted to change from the first state to the second state when a pre-determined strain is placed on the releasing member 801. The strain preferably being caused by an abnormal movement of the hip joint, e.g. as the result of the patient falling. According to the embodiment shown in fig. 9 the releasing member 801 comprises an elastic portion comprising elastic material, in the embodiment shown being the entire releasing member 801. The releasing member is adapted to non-invasively be able to change from the first state to the second state and from the second state to the first state, when a pre-determined strain is placed on the releasing member 801.

Fig. 10 shows the hip joint in section when the releasing member 801 is in its second state, wherein the releasing member 801 is adapted to release the caput femur 5, or an artificial replacement therefore, from the artificial bowl shaped acetabulum cup 65 placed in the pelvic bone 9. The releasing member 801 has changed from the first state to the second state because of a pre-determined strain has been placed on the releasing members 801.

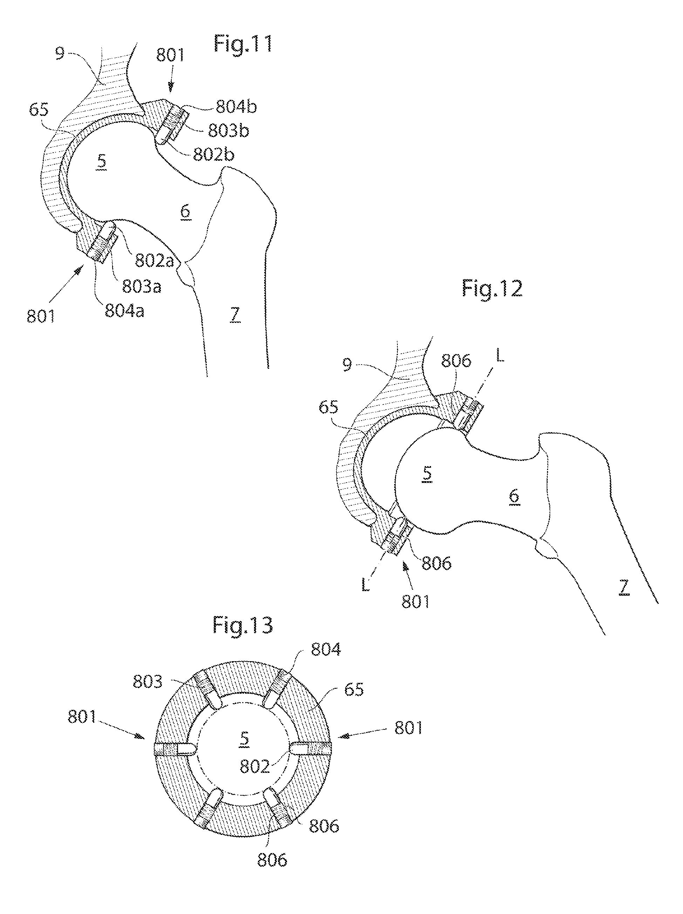

Fig. 11 shows the medical device according to an embodiment where the artificial bowl shaped acetabulum surface 65 comprises releasing members 801 comprising holding members 802a,b adapted to slide against the caput femur 5, or an artificial replacement therefore. The holding members are adapted to, in a first state, hold the caput femur 5, or an artificial replacement therefore, which is a ball shaped part attached to the collum femur 6 in position in the hip joint to the artificial bowl shaped acetabulum cup 65 placed in the pelvic bone 9. In a second state the releasing member 801 is adapted to release the caput femur 5, or an artificial replacement therefore, from the artificial bowl shaped acetabulum cup 65 placed in the pelvic bone 9. The holding members 802a,b are spring loaded through a spring 803a,b being placed between a calibration member, being a calibration screw 804a,b, and the holding members 802a,b. The force exerted on the holding members 802a,b from the spring 803a,b is adapted to hold the caput femur 5, or an artificial replacement therefore, in the artificial acetabulum 65 in normal, functional hip joint movements, but release the caput femur 5, or an artificial replacement therefore, from the artificial acetabulum 65 when a pre-determined strain is placed on the releasing member preferably being caused by an abnormal movement of the hip joint, e.g. as the result of the patient falling. The calibration screws 804a,b enables the pre-determination of the strain which will cause the holding members 802a,b to change from being in a first state to being in a second state.

Fig 12 shows the releasing members in their second state, when a pre-determined strain has been exceeded, preferably being caused by an abnormal movement of the hip joint, e.g. as the result of the patient falling. The holding members 802a,b are retracted into sleeves 806 of the artificial acetabulum surface 65, thereby compressing the springs 803a,b. The retraction of the holding members 802a,b causes the caput femur 5, or an artificial replacement therefore, to be dislocated/luxated from its position in the artificial acetabulum surface 65, which, when large strain is placed on the hip joint and femoral bone 7, reduces the risk of the patient fracturing the femoral bone 7 or the pelvic bone 9. The holding members 802a,b are adapted to non-invasively be able to change from the first state to the second state and from the second state to the first state, when a pre-determined strain is placed on the holding members 802a,b.

Fig. 13 shows the artificial acetabulum 65 in section with the holding members 802, placed in sleeves 806 evenly distributed along the cross-section of the artificial acetabulum 65, holding the caput femur 5, or an artificial replacement therefore, in position in the artificial acetabulum 65.

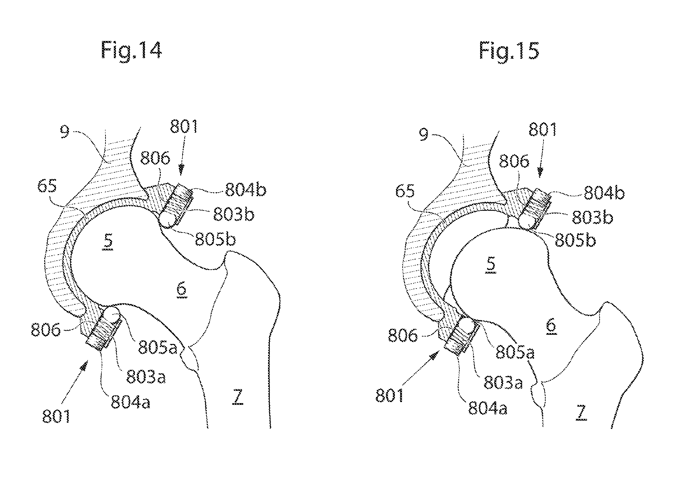

Fig. 14 shows an alternative embodiment of the principle shown in figs 11-13, wherein the holding members 802a,b, comprises ball shaped members 805a,b in contact with the caput femur 5, or an artificial replacement therefore, ant being adapted to roll against the caput femur 5, or an artificial replacement therefore, holding the caput femur 5, or an artificial replacement therefore, in place in the artificial acetabulum 65 by the holding members 802a,b exerting force on the caput femur 5, or an artificial replacement therefore, through the contact with the springs 803a,b supported by the calibration screws 804a,b.

Fig 15 shows the releasing members in their second state, when a pre-determined strain has been exceeded, preferably being caused by an abnormal movement of the hip joint, e.g. as the result of the patient falling. The holding members 802a,b, comprising the ball shaped members 805a,b, are retracted into sleeves 806 of the artificial acetabulum surface 65, thereby compressing the springs 803a,b. The retraction of the holding members 802a,b causes the caput femur 5, or an artificial replacement therefore, to be dislocated/luxated from its position in the artificial acetabulum surface 65, which, when large strain is placed on the hip joint and femoral bone 7, reduces the risk of the patient fracturing the femoral bone 7 or the pelvic bone 9. The holding members 802a,b are adapted to non-invasively be able to change from the first state to the second state and from the second state to the first state, when a pre-determined strain is placed on the holding members 802a,b, which enables the caput femur 5, or an artificial replacement therefore, to be replaced in the artificial acetabulum 65 without a surgical procedure.

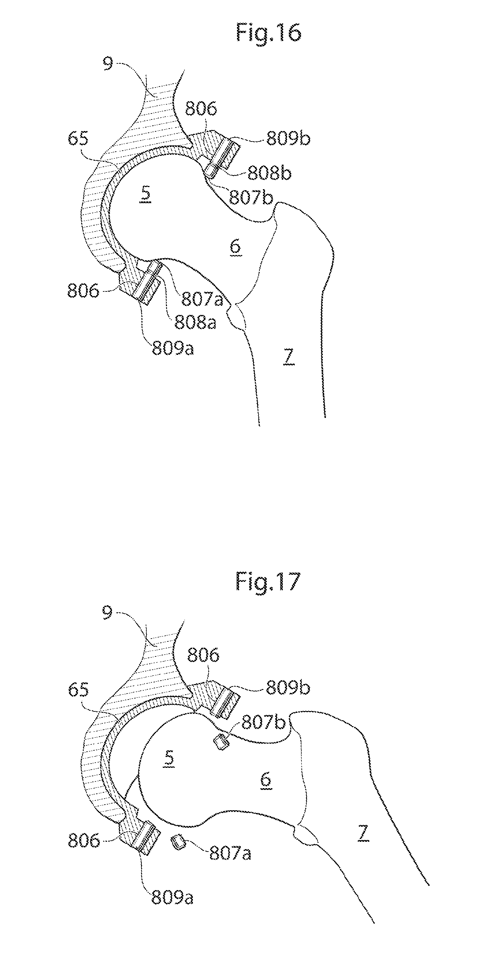

Fig 16 shows the medical device in an embodiment wherein the releasing members 801 comprises a rupture device 807, 808, 809 adapted to fail at a pre-determined strain. According to this embodiment the rupture device is a rupture pin 807, 808, 809 comprising a base part 809a,b fixated to the artificial acetabulum 65 and a rupture part 807a,b attached to the base part 809a,b through a weakened section 808a,b, in which section the rupture part 807a,b is detached from the base part 809a,b when a predetermined strain is placed on the rupture device in contact with the caput femur 5, or an artificial replacement therefore.

Fig. 17 shows the medical device according to the embodiment of fig. 16 when the rupture device has failed due to a pre-determined strain on the rupture device being exceeded. According to one embodiment, (not shown) the rupture parts 807a,b are secured to the base part through a security wire keeping rupture parts 807a,b in proximity to the base part 809a,b even after the failure of the rupture device.

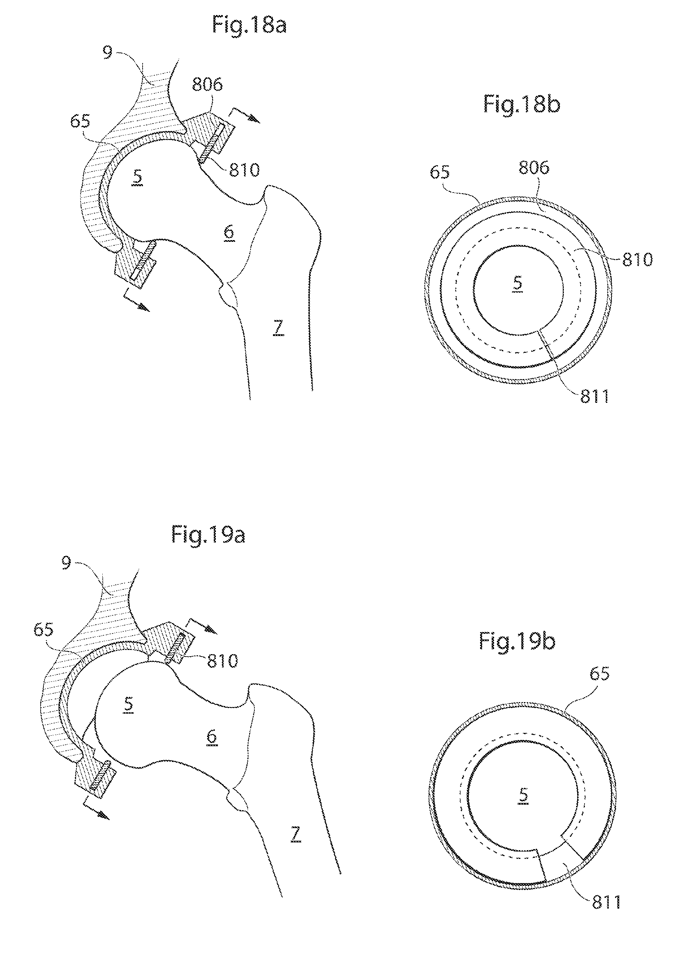

Fig 18a shows the medical device according to an embodiment where the artificial acetabulum 65 comprises a circular sleeve 806, in which an elastic or rupture band 810 is provided. The elastic or rupture band 810 is adapted to at least partly encircle the ball shaped caput femur 5, or artificial replacement therefore. When a pre-determined strain is placed on the elastic or rupture band 810 the circular opening encircling the caput femur 5, or an artificial replacement therefore, is expanded and the caput femur 5, or an artificial replacement therefore, is released from the artificial acetabulum 65, to which it is held by means of the elastic band 610. In embodiments where the medical device comprises a rupture band 810 holding the caput femur 5, or an artificial replacement therefore, in the artificial acetabulum 65, a weakened portion 811 of the band 810 fails and thus the circular opening encircling the caput femur 5, or an artificial replacement therefore, is expanded and the caput femur 5, or an artificial replacement therefore, is released from the artificial acetabulum 65. In the embodiments where the band 810 is an elastic band 810 it is conceivable that the band 810 comprises an elastic part or section, or that the entire band 810 is made of an elastic material.

Fig. 18 b shows the medical device in section when the elastic or rupturing band 810, holding the caput femur 5, or an artificial replacement therefore, is placed in a circular sleeve 806 in the artificial acetabulum 65. An opening or weakened portion 811 is provided perpendicular to the circumference of the band 810.

Fig. 19a shows the medical device in a second state where the caput femur 5, or an artificial replacement therefore, is released from the connection with the acetabulum, after a pre-determined stain has been placed on the elastic or rupture band 810. As shown in fig. 19b the gap or weakened part has been expanded, thereby allowing the caput femur, or an artificial replacement therefore, 5 to pass through the opening defined by the elastic or rupture band 810. The medical device could be adapted to non-invasively be able to change from the first state to the second state and from the second state to the first state, when a pre-determined strain is placed on the band 810, which enables the caput femur 5, or an artificial replacement therefore, to be replaced in the artificial acetabulum 65 without a surgical procedure.

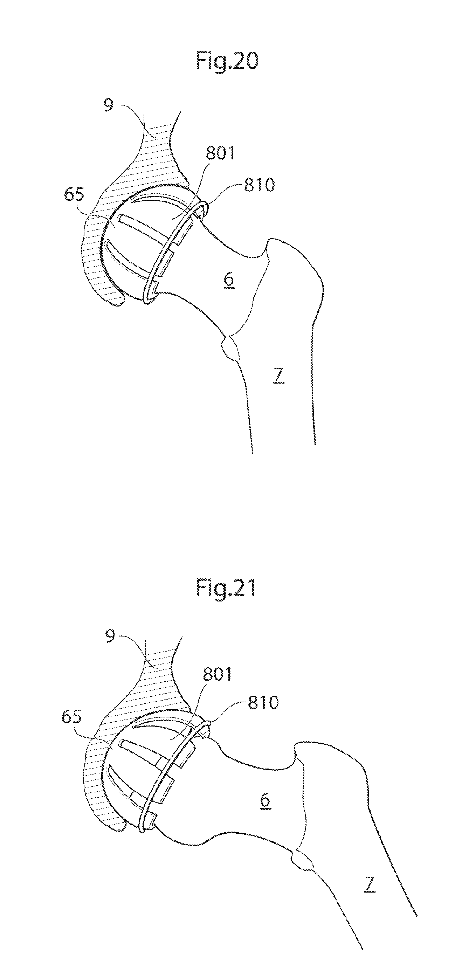

Fig. 20 shows the medical device according to an embodiment where the releasing member 801 comprises an elastic wing of the artificial acetabulum 65, which is assisted by an elastic or rupture band 810 encircling the medical device by enclosing the caput femur 5, or an artificial replacement therefore, in the artificial acetabulum 65 passing beyond the point of the caput femur 5, or an artificial replacement therefore, having a largest cross-sectional distance. The elastic or rupture band 810 is held in place to the artificial acetabulum 65 by means of the band 810 being placed in a groove along the circumference of the artificial acetabulum 65. However, said groove could be assisted or replaced by an adhesive or a mechanical fixation element.

Fig. 21 shows the medical device when in its second state, in which the releasing member 801 releases the caput femur 5, or an artificial replacement therefore, from the artificial acetabulum 65. In embodiments when the band 810 is an elastic band 810 it is expanded, thereby enlarging the hole through which the caput femur 5, or an artificial replacement therefore, can pass. In embodiment where the band 810 is a rupture band, the band 810 has failed and thereby the caput femur 5, or an artificial replacement therefore, is held in place solely by the releasing member 801 which is adapted to release the caput femur 5, or an artificial replacement therefore, at a pre-defined strain. The medical device could be adapted to non-invasively be able to change from the first state to the second state and from the second state to the first state, when a pre-determined strain is placed on the band 810 and/or the releasing member 801, which enables the caput femur 5, or an artificial replacement therefore, to be replaced in the artificial acetabulum 65 without a surgical procedure.

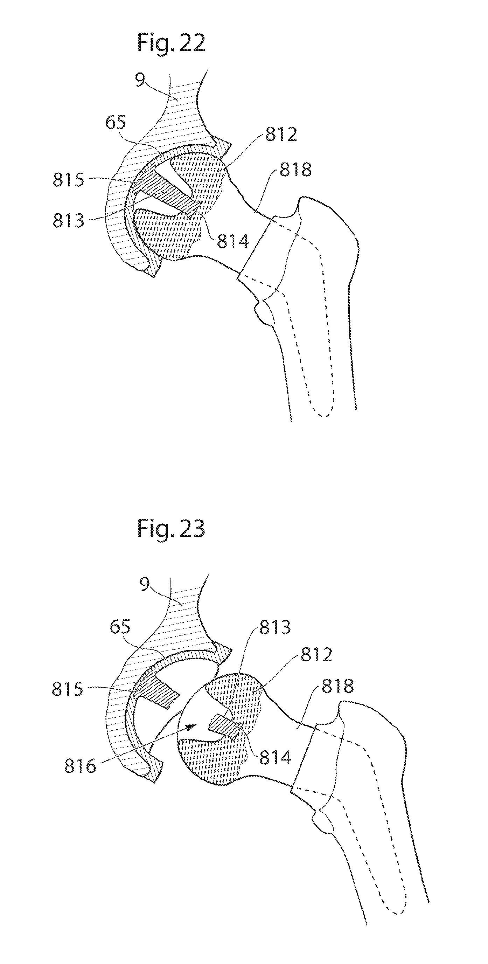

Fig. 22 shows the hip joint in section according to an embodiment where the caput femur 5, or an artificial replacement therefore, and collum femur 6 have been replaced with a prosthetic part 818 fixated to the femoral bone 7, either with bone cement, or without. The prosthetic part 818 comprises an artificial caput femur 812 having a cavity 816 in which a rupture band 813 fixated to a fixation portion 814 of the artificial caput femur 812, and a fixating portion 815 of the artificial acetabulum 65. The cavity 816 is adapted to enable the artificial caput femur 812 to perform normal functional hip movements inside the artificial acetabulum 65. The rupture band 813 is adapted to hold the artificial caput femur 812 to the artificial acetabulum 65 in a first state, and release the artificial caput femur 812 from the artificial acetabulum when a pre-determined strain is placed on the rupture band 813.

Fig. 23 shows the embodiment of the medical device according to fig. 22, in a second state in which the rupture band 813 has failed and thereby the artificial caput femur 812 is released from the artificial acetabulum 65. The rupture band 813 could be fixated to a fixation portion 814 of the artificial caput femur 812, and/or a fixating portion 815 of the artificial acetabulum 65 using: at least one screw, at least one pin, form fitting, welding, adhesive, pin, wire, a ball mounted into a bowl, a male portion of one part mounted into a female portion of the other part, a key introduced into a lock being portions of said parts, band, or other mechanical connecting members. The failing of the rupture band 813 is preferably caused by an abnormal movement of the hip joint, e.g. as the result of the patient falling.

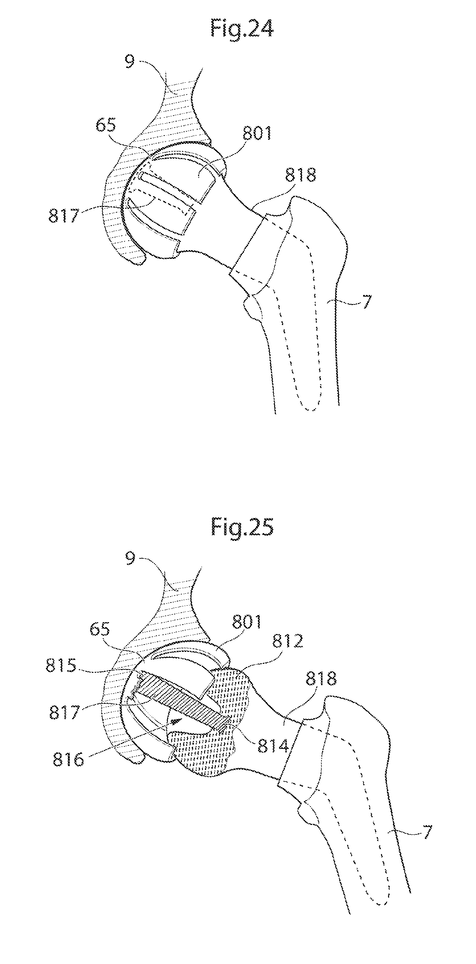

Fig. 24 shows a prosthetic part 818 according to an embodiment where the prosthetic part 818 is fixated to the femoral bone 7 and comprises a caput femur 812 comprising a cavity 816 adapted to enable the hip joint to perform functional hip joint movements while in a first state held to the artificial acetabulum using an elastic bend 817 fixated to a fixation portion 814 of the artificial caput femur 812, and a fixating portion 815 of the artificial acetabulum 65, and a releasing member 801 according to the embodiment shown in figs. 9 and 10. The combination of the releasing member 801 and the elastic band 817 is adapted to, in a first state hold the prosthetic part 818 to the artificial acetabulum 65, and in a second state release the prosthetic part 818 from the artificial acetabulum 65. According to another embodiment (not shown) the prosthetic part is held to the artificial acetabulum 65 solely using the elastic band 817, of course also supported by the remainder of the hip joint capsule and the affected muscles.

Fig. 25 shows the embodiment of the medical device according to fig. 24, in a second state in which the elastic band 817 is stretched such that the prosthetic part 818 is released from the artificial acetabulum artificial acetabulum 65. The elastic band 817 could be fixated to a fixation portion 814 of the artificial caput femur 812, and/or a fixating portion 815 of the artificial acetabulum 65 using: at least one screw, at least one pin, form fitting, welding, adhesive, pin, wire, a ball mounted into a bowl, a male portion of one part mounted into a female portion of the other part, a key introduced into a lock being portions of said parts, band, or other mechanical connecting members. The failing of the rupture band 813 is preferably caused by an abnormal movement of the hip joint, e.g. as the result of the patient falling. Preferably the elastic band 817 comprises an elastic part or section, which could be the entire elastic band 818, made from an elastic material, such as an elastic polymer material such as: a copolymer material such as polystyrene, poly(ethylene-butylene) or polystyrene. It is also conceivable that the material is a polyurethane elastomeric material, polyamide elastomeric materials and polyester elastomeric materials elastic copolymers of ethylene and at least one vinyl monomer such as, for example, vinyl acetates, unsaturated aliphatic monocarboxylic acids, and esters of such monocarboxylic acids. The elastic band 813 could comprise a barrier coating, which cannot be penetrated by body cells. Preferably, the barrier coating comprises a Parylene™ coating, or a biocompatible metal coating, such as gold, silver or titanium. According to other embodiments the elastic band comprises a spring type member, a combination of metal and plastic materials, a combination of metal and carbon based material or a combination of carbon and plastic based material.

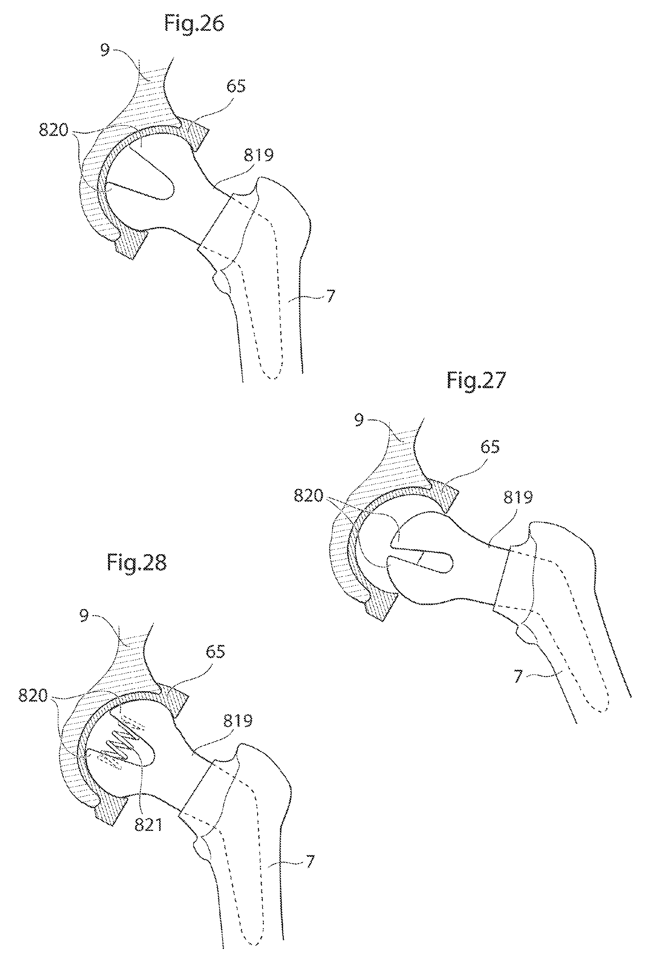

Fig. 26 shows the hip joint in section in an embodiment where the medical device comprises a prosthetic part 819 adapted to be fixated to the femoral bone 7. The prosthetic part comprises an artificial caput femur which is adapted to comprise elastic elements 820 which act as a releasing member holding the artificial caput femur inside of the artificial acetabulum 65 fixated to the pelvic bone. The elastic elements 820 of the artificial caput femur, is preferably made of an elastic material, which for example could be an elastomeric polymer material or an elastic metal material. It is conceivable that the elastic material comprises an outer layer in connection with the artificial acetabulum 65 which is adapted to resist the wear from the contact with the artificial acetabulum surface. The elastic element is adapted to compress when a pre-determined strain is placed on the hip joint and thereby on the elastic elements 820. When the elastic elements 820 are compressed the artificial caput femur is released from the artificial acetabulum 65.

Fig. 27 shows the medical device according to the embodiment shown in fig. 27, in a second state, in which the elastic element 820 has been compressed, following a pre-determined strain being placed on the medical device. The medical device is thereby placed in a second state, in which the artificial caput femur is released from the artificial acetabulum 65, wherein it has been held.

Fig. 28 shows an embodiment of the medical device in which the elastic elements 820 are further assisted by a spring 821 in connection with two elastic elements 820, the spring 821 is compressed alongside the elastic members 820, when a pre-determined strain is placed on the prosthetic part 819 comprising the artificial caput femur.

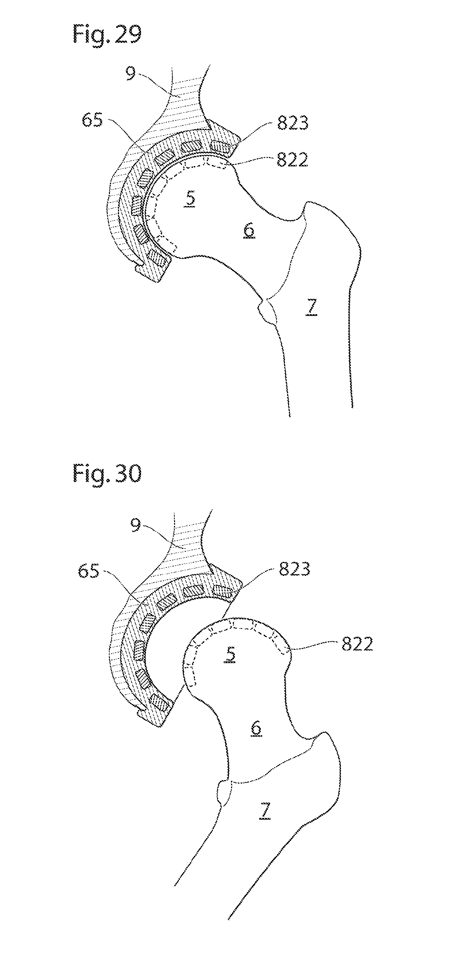

Fig. 29 shows the hip joint in section when a medical device for, in a first state, holding the caput femur 5, or an artificial replacement therefore, to the artificial acetabulum 65, and in a second state releasing the caput femur 5, or an artificial replacement therefore from the artificial acetabulum 65. The medical device is adapted to change from being in the first state to being in the second state at a pre-determined strain affecting the medical device by the connection with the pelvic bone 9 and the femoral bone 7, which reduced the risk of the patient fracturing the femoral bone 7 and/or the pelvic bone 9. The medical device comprises magnets 823 or magnetic material 823 placed in the artificial acetabulum 65, and magnets 822 or magnetic material 822 placed in the caput femur 5 or an artificial replacement therefore. According to one embodiment a magnet 823 is placed in the artificial acetabulum having its south pole directed towards the caput femur 5, or artificial replacement therefore, and a magnet 822 placed in the caput femur 5, or artificial replacement therefore, having its north pole directed towards the artificial acetabulum 65. However it is easily understood by the skilled in the art that only one of the sides needs to be magnetic whereas the other side merely needs to comprise magnetic material. Any combination of north and south ends and magnets/magnetic material is hence conceivable. The magnetic force described is adapted to hold the caput femur 5, or an artificial replacement therefore, in the acetabulum in normal use, enabling the hip joint to perform functional hip joint movements, and release the caput femur 5, or an artificial replacement therefore, from the artificial acetabulum 65 when a predetermined strain is exceeded.

Fig 30 shows the medical device according to the embodiment of fig. 30 in the second state, in which the caput femur 5, or an artificial replacement therefore, is released from the artificial acetabulum 65 as a result of a pre-determined level of strain being exceeded.

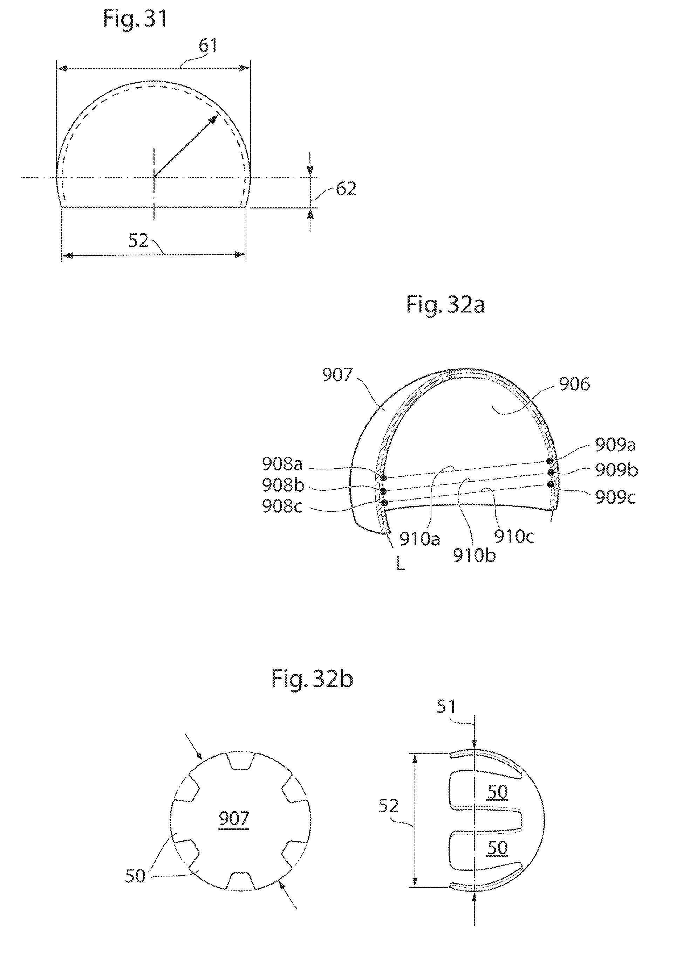

Fig. 31 shows, schematically, how the artificial acetabulum travels beyond the maximum diameter of the caput femur 5, or an artificial replacement therefore. That is, a cross-sectional distance of the largest opening 52 is smaller than the largest cross sectional distance of the caput femur 5 or an artificial replacement therefore

Fig. 32 shows the medical device according to an embodiment in which the second piece comprises: an inner surface 906, and an outer surface 907. The inner surface 906 comprises: a first point 908a, a second point 909a, a third point 908b, a fourth point 909b, a fifth point 908c, and a sixth point 909c, all points located on different places along a length axis of the inner surface. A first straight line 910a, reaches from the first point 908a to the second 909a and is parallel to a second straight 910b line reaching from the third point 908b to the fourth point 909b, which in turn is parallel to a third straight 910c line, reaching from the fifth point 908c to the sixth point 909c. The first 910a and the third 910c straight lines are shorter than the second straight line, and the second straight line is positioned between the first and third straight lines.

### Opposite embodiment

A general version of an opposite embodiment will now be described, the scope of the opposite embodiment is by no means limited to this particular version, on the contrary all of the above described embodiment can be used in the opposite embodiment.

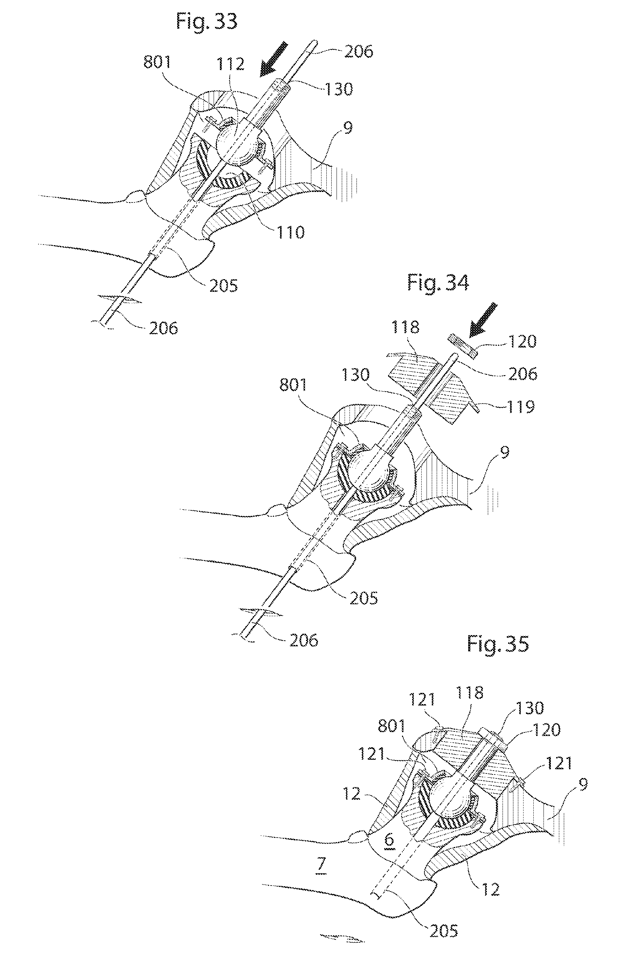

Fig. 33 shows the hip joint in section when an artificial caput femur surface 112 is fixated to a surgically modified caput femur comprising a concave artificial acetabulum surface 110 placed in the surgically modified caput femur. According to the embodiment shown in fig. 33 an elongated member 206 is used as a guiding rod, guiding and centering the artificial acetabulum surface, and the artificial caput femur surface in the hip joint. The convex hip joint surface 112 is secured by the releasing member 801 which is adapted to, in a first state, hold the artificial caput femur, and in a second state release the artificial caput femur, and change from a first to a second state when a pre-determined strain is exceeded. The releasing member is fixated to the surgically modified caput femur using screws 121. The surface of the locking element 117 and the concave hip joint surface 117 is placed in connection with the convex hip joint surface and could be made of a friction reducing material such as PTFE or a self lubricating powder material. However it is also conceivable that the connecting surfaces are lubricated using an implantable lubrication system adapted to lubricate the medical device after said medical device has been implanted in the human patient, a solution conceivable in all of the above described embodiments. According to the embodiment shown the elongated member 206 is inserted through the femoral bone, however according to other embodiments, not shown, the elongated member is positioned inside of the hip joint from the acetabulum side.

Fig. 34 shows the placing of a prosthetic part 118 adapted to occupy the hole 18 created in the pelvic bone 9. The prosthetic part 118 comprises supporting members 119 adapted to be in contact with the pelvic bone 9 and assist in the carrying of the load placed on the medical device from the weight of the human patient. Furthermore fig. 34 shows the fixation of a nut 120 to the attachment rod 113, which in turn is guided by the elongated member 206 which acts as a guiding rod.

Fig. 35 shows the hip joint in section when all the elements of the medical device has been fixated in the area of the hip joint or its surroundings. The prosthetic part 113 adapted to occupy the hole 18 in the pelvic bone 9 is here fixated with screws 121, however these screws 121 could be assisted or replaced by an adhesive which could be applied to the surface S between the prosthetic part and the pelvic bone 9. The elongated member 206 which acts as a guiding rod has been retracted through the incision in the thigh.

The elastic or flexible part, piece or portion of any of the embodiments herein could comprise an elastic polymer material such as: a copolymer material such as polystyrene, poly(ethylenebutylene) or polystyrene. It is also conceivable that the material is a polyurethane elastomeric material, polyamide elastomeric materials and polyester elastomeric materials elastic copolymers of ethylene and at least one vinyl monomer such as, for example, vinyl acetates, unsaturated aliphatic monocarboxylic acids, and esters of such monocarboxylic acids. The elastic band 813 could comprise a barrier coating, which cannot be penetrated by body cells. Preferably, the barrier coating comprises a Parylene™ coating, or a biocompatible metal coating, such as gold, silver or titanium. According to other embodiments the elastic band comprises a spring type member, a combination of metal and plastic materials, a combination of metal and carbon based material or a combination of carbon and plastic based material.

The artificial acetabulum, according to any of the embodiments, could comprise one or more parts which could be fixated to the pelvic bone using at least one screw, at least one pin, at least one portion of at least one of the parts adapted to be introduced into the other part, the parts being adapted to be sliding into the other part, form fitting, welding, adhesive, pin, wire, a ball mounted into a bowl being portions of said parts, a male portion of one part mounted into a female portion of the other part, a key introduced into a lock being portions of said parts, band, or other mechanical connecting members.

Please note that any embodiment or part of embodiment as well as any method or part of method could be combined in any way. All examples herein should be seen as part of the general description and therefore possible to combine in any way in general terms.

## Claims

1. A medical device for reducing the risk of permanent damage to at least one of; the femoral bone (7), the pelvic bone (9), components of a prosthetic hip joint, and a fixation between a prosthetic hip joint and at least one of the fernoral bone and the pelvic bone, said medical device comprises a first and second piece, and a releasing member (801) adapted to, in a first state hold said first piece attached to said second piece, and in a second state release said first piece from said second piece, and wherein said releasing member is adapted to change from said first state to said second state when a pre-determined strain is placed on said releasing member, **characterized in that** said medical device further comprises a calibration member (804) for calibrating the pre-determined strain required for said releasing member to change from said first state to said second state.

2. The medical device according to claim 1, wherein said calibration member is a calibration screw.

3. The medical device according to any one of the preceding claims, wherein said first piece comprises a ball shaped piece (812), adapted to replace at least the surface of the caput femur (5) of the hip joint, and/or the second piece comprises a bowl shaped piece (65), adapted to replace at least the acetabulum, surface (11) of the hip joint.

4. The medical device according to claim 3, wherein said ball shaped piece is adapted to be fixated in said bowl shaped piece using said releasing member.

5. The medical device according to claim 3 or 4, wherein at least one of:
a. said ball shaped piece of said medical device comprises said releasing member
b. said second piece of said medical device comprises said releasing member, and
c. said bowl shaped piece of said medical device comprises said releasing member.

6. The medical device according to any one of the preceding claims, wherein said releasing member comprises an elastic portion.

7. The medical device according to any one of the preceding claims, wherein said releasing member comprises a magnet (822; 823) adapted to hold said first piece to said second piece.

8. The medical device according to any one of the preceding claims, wherein said releasing member comprises an elastic band (813; 810).

9. The medical device according to claim 8, wherein said elastic band is adapted to at least one of:
a. at least partly encircle said ball shaped piece, and
b. be placed between said ball shaped piece and said bowl shaped piece.

10. The medical device according to any one of the preceding claims, wherein said releasing member comprises a rupture device (813; 807; 808; 809) adapted to fail at a pre-determined strain.

11. The medical device according to any one of the preceding claims, wherein said releasing member comprises multiple holding members (802; 805).

12. The medical device according to claim 11, wherein said multiple holding members are adapted to at least one of:
a. slide against said first piece, and
b. roll against said first piece.

13. The medical device according to any of the preceding claims, wherein the releasing member comprises a locking member for locking the second piece being an artificial replacement of an acetabulum in a hip joint to clasp the first piece being an artificial replacement of caput femur, when implanted in a hip joint of a patient, wherein said locking member is adapted to in situ assist in the fixation of the medical device, wherein: said locking member is adapted to lock said artificial replacement of caput femur such that it remains clasped and restrained in said inner surface until released by said releasing member, and said locking member is adapted to lock said at least one extension portion, when implanted, having at least the end portion of the extension portion radially fixed within said circular extension line, and wherein said locking member is adapted to lock in at least a first and second locking position.

14. The medical device according to claim 13, wherein said locking member is adapted to lock in at least a first and a second locking position, and wherein said locking member is adapted to; in said first locking position, lock the artificial acetabulum inner surface having at least one extending portion, to a first size artificial caput femur, and in said second locking position, lock said artificial actabulum inner surface, to a second smaller size artificial caput femur.

15. The medical device according to any one of claims 13 and 14, wherein at least one extending portion is adapted to have a shape or position such that the restriction of movement range of the hip joint, in degrees from maximal movement, is restricted more in at least one predefined direction than in any other direction, when implanted.

## Patentansprüche

1. Medizinische Vorrichtung zum Verringern der Gefahr eines dauerhaften Schadens für mindestens einen des Oberschenkelknochens (7), des Beckenknochens (9), Bestandteilen eines prothetischen Hüftgelenks und einer Befestigung zwischen einem prothetischen Hüftgelenk und mindestens einem des Oberschenkelknochens und de Beckenknochens, wobei die medizinische Vorrichtung ein erstes und ein zweites Teil aufweist sowie ein Freigabeelement (801), das dazu eingerichtet ist, in einem ersten Zustand das erste Teil, das mit dem zweiten Teil verbunden ist, zu halten und in einem zweiten Zustand das erste Teil von dem zweiten Teil zu lösen, und wobei das Freigabeelement dazu eingerichtet ist, von dem ersten Zustand in den zweiten Zustand zu wechseln, wenn eine vorgegebene Belastung auf das Freigabeelement erfolgt, **dadurch gekennzeichnet, dass** die medizinische Vorrichtung ferner ein Kalibrierungselement (804) zum Kalibrieren der vorgegebenen Belastung aufweist, welches für das Freigabeelement erforderlich ist, um von dem ersten Zustand in den zweiten Zustand zu wechseln.

2. Medizinische Vorrichtung nach Anspruch 1, wobei das Kalibrierungselement eine Kalibrierungsschraube ist.

3. Medizinische Vorrichtung nach einem der vorhergehenden Ansprüche, wobei das erste Teil ein ballförmiges Teil (812) aufweist, das dazu eingerichtet ist, mindestens die Oberfläche des Caput Femurs (5) des Hüftgelenks zu ersetzten, und/oder das zweite Teil ein schalenförmiges Teil (65) aufweist, das dazu eingerichtet ist, mindestens die Fläche (11) der Hüftgelenkspfanne des Hüftgelenks zu ersetzen.

4. Medizinische Vorrichtung nach Anspruch 3, wobei das ballförmige Teil dazu ausgebildet ist, in dem schalenförmigen Teil unter Verwerdung des Freigabeelements befestigt zu werden.

5. Medizinische Vorrichtung nach Anspruch 3 oder 4, wobei mindestens eines
a. des ballförmigen Teils der medizinischen Vorrichtung das Freigabeelement aufweist,
b. des zweiten Teils der medizinischen Vorrichtung das Freigabeelement aufweist, und
c. des schalenförmigen Teils der medizinischen Vorrichtung das Freigabeelement aufweist.

6. Medizinische Vorrichtung nach einem der vorhergehenden Ansprüche, wobei das Freigabeelement einen elastischen Abschnitt aufweist.

7. Medizinische Vorrichtung nach einem der vorhergehenden Ansprüche, wobei das Freigabeelement einen Magneten (822; 823) aufweist, der dazu ausgebildet ist, das erste Teil an dem zweiten Teil festzuhalten.

8. Medizinische Vorrichtung nach einem der vorhergehenden Ansprüche, wobei das Freigabeelement ein elastisches Band (813; 810) aufweist.

9. Medizinische Vorrichtung nach Anspruch 8, wobei das elastische Band für mindestens eines des Folgenden ausgebildet ist:
a. mindestens teilweise das ballförmigen Teil zu umgeben, und
b. zwischen dem ballförmigen Teil und dem schalenförmigen Teil angeordnet zu werden.

10. Medizinische Vorrichtung nach einem der vorhergehenden Ansprüche, wobei das Freigabeelement eine Bruchvorrichtung (813; 807; 808; 809) aufweist, die dazu ausgebildet ist, bei einer vorgegebenen Belastung zu brechen.

11. Medizinische Vorrichtung nach einem der vorhergehenden Ansprüche, wobei das Freigabeelement mehrere Halteglieder (802; 805) aufweist.

12. Medizinische Vorrichtung nach Anspruch 11, wobei die mehreren Halteglieder für mindestens eines des Folgenden ausgebildet sind:
a. gegen das erste Teil zu gleiten, und
b. gegen das erste Teil zu rollen.

13. Medizinische Vorrichtung nach einem der vorhergehenden Ansprüche, wobei das Freigabeelement ein Sicherungsglied zum Sichern des zweiten Teils aufweist, das ein künstlicher Ersatz einer Hüftgelenkspfanne in einem Hüftgelenk ist, um das erste Teil, das ein künstlicher Ersatz eines Caput Femurs ist, zu umklammern, wenn es in einem Hüftgelenk eines Patienten eingesetzt wird, wobei das Sicherungsglied dazu ausgebildet ist, in situ bei der Befestigung der medizinischen Vorrichtung unterstützend zu wirken, wobei das Sicherungsglied dazu ausgebildet ist, den künstlichen Ersatz des Caput Femur zu sichern, so dass es in der inneren Fläche umklammert und zurückgehalten wird, bis es von dem Freigabeelement gelöst wird, und wobei das Sicherungsglied dazu ausgebildet ist, nach dem Einsetzen den mindestens einen Verlängerungsabschnitt zu sichern, wobei mindestens der Endabschnitt des Verlängerungsabschnitts radial innerhalb der kreisförmigen Verlängerungslinie befestigt ist, und wobei das Sicherungselement dazu ausgebildet ist, in mindestens einer ersten und zweiten Sicherungsposition zu arretieren.

14. Medizinische Vorrichtung nach Anspruch 13, wobei das Sicherungselement dazu ausgebildet ist, in mindestens einer ersten und zweiten Sicherungsposition zu arretieren, und wobei das Sicherungselement dazu ausgebildet ist: in der ersten Sicherungsposition die Innenfläche der künstlichen Hüftgelenkspfanne, die mindestens einen Verlängerungsabschnitt aufweist, an einem künstlichen Caput Femur erster Größe zu fixieren, und in der zweiten Sicherungsposition die Innenfläche der künstlichen Hüftgelenkspfanne an einem kleineren künstlichen Caput Femur zu fixieren.

15. Medizinische Vorrichtung nach einem der Ansprüche 13 und 14, wobei mindestens ein Verlängerungsabschnitt dazu ausgebildet ist, nach dem Einsetzen eine Form oder Position aufzuweisen, so dass die Einschränkung des Bewegungsbereichs des Hüftgelenks in Grad der maximalen Bewegung mehr in mindestens eine vorgegebene Richtung als in eine andere Richtung begrenzt ist.

## Revendications

1. Dispositif médical destiné à réduire le risque de dommage permanent à au moins : l'os (7) fémoral, l'os (9) pelvien, les composants d'une prothèse de hanche, et/ou une fixation entre une prothèse de hanche et ledit au moins l'os fémoral et l'os pelvien, ledit dispositif médical comportant une première et une seconde pièce, et un élément (801) de libération conçu, dans un premier état, pour maintenir ladite première pièce fixée à ladite seconde pièce, et dans un second état, pour libérer ladite première pièce de ladite seconde pièce, et
ledit élément de libération étant conçu pour changer dudit premier état audit second état lorsqu'une contrainte préétablie est placée sur ledit élément de libération, **caractérisé en ce que** ledit dispositif médical comporte en outre un élément (804) d'étalonnage destiné à étalonner la contrainte préétablie requise pour que ledit élément de libération change dudit premier état audit second état.

2. Dispositif médical selon la revendication 1, ledit élément d'étalonnage étant une vis d'étalonnage.

3. Dispositif médical selon l'une quelconque des revendications précédentes, ladite première pièce comportant une pièce (812) de forme sphérique, conçue pour remplacer au moins la surface de la tête de fémur (5) de l'articulation de la hanche, et/ou la seconde pièce comportant une pièce (65) en forme de cuvette, conçue pour remplacer au moins la surface (11) de l'acétabulum de l'articulation de la hanche.

4. Dispositif médical selon la revendication 3, ladite pièce de forme sphérique étant conçue pour être fixée dans ladite pièce en forme de cuvette à l'aide dudit élément de libération.

5. Dispositif médical selon la revendication 3 ou 4, au moins :
a. ladite pièce de forme sphérique dudit dispositif médical comportant ledit élément de libération
b. ladite seconde pièce dudit dispositif médical comportant ledit élément de libération, et
c. ladite pièce en forme de cuvette dudit dispositif médical comportant ledit élément de libération.

6. Dispositif médical selon l'une quelconque des revendications précédentes, ledit élément de libération comportant une partie élastique.

7. Dispositif médical selon l'une quelconque des revendications précédentes, ledit élément de libération comportant un aimant (822 ; 823) conçu pour maintenir ladite première pièce et ladite seconde pièce ensemble.

8. Dispositif médical selon l'une quelconque des revendications précédentes, ledit élément de libération comportant une bande (813 ; 810) élastique.

9. Dispositif médical selon la revendication 8, ladite bande élastique étant conçue pour au moins :
a. entourer au moins partiellement ladite pièce de forme sphérique, et/ou
b. être placée entre ladite pièce de forme sphérique et ladite pièce en forme de cuvette.

10. Dispositif médical selon l'une quelconque des revendications précédentes, ledit élément de libération comportant un dispositif (813 ; 807 ; 808 ; 809) de rupture conçu pour échouer à une contrainte préétablie.

11. Dispositif médical selon l'une quelconque des revendications précédentes, ledit élément de libération comportant de multiples éléments (802 ; 805) de maintien.

12. Dispositif médical selon la revendication 11, lesdits multiples éléments de maintien étant conçus pour au moins :
a. glisser contre ladite première pièce, et
b. rouler contre ladite première pièce.

13. Dispositif médical selon l'une quelconque des revendications précédentes, l'élément de libération comportant un élément de verrouillage destiné à verrouiller la seconde pièce qui est un substitut artificiel d'un acétabulum dans une articulation de la hanche pour enserrer la première pièce qui est un substitut artificiel de la tête du fémur, lorsqu'implanté dans une articulation de la hanche d'un patient,
ledit élément de verrouillage étant conçu pour faciliter la fixation in situ du dispositif médical : ledit élément de verrouillage étant conçu pour verrouiller ledit substitut artificiel de la tête de fémur de manière à ce qu'il reste enserré et retenu dans ladite surface interne jusqu'à sa libération par ledit élément de libération, et ledit élément de verrouillage étant conçu pour verrouiller au moins ladite partie allongée, lorsqu'il est implanté, ayant au moins la partie d'extrémité de la partie allongée fixée radialement à l'intérieur de ladite ligne allongée circulaire, et ledit élément de verrouillage étant conçu pour se verrouiller dans au moins une première et une seconde position de verrouillage.

14. Dispositif médical selon la revendication 13, ledit élément de verrouillage étant conçu pour se verrouiller dans au moins une première et une seconde position de verrouillage, et ledit élément de verrouillage étant conçu, dans ladite première position de verrouillage, pour verrouiller la surface interne de l'acétabulum artificielle qui a au moins une partie s'étendant sur une tête de fémur artificielle d'une première taille, et dans ladite seconde position de verrouillage, verrouiller ladite surface interne de l'acétabulum artificielle, sur une tête de fémur artificielle d'une seconde taille plus petite.

15. Dispositif médical selon l'une quelconque des revendications 13 et 14, ladite au moins une partie allongée étant conçue pour avoir une forme ou une position telle que la restriction de la plage de mouvement de l'articulation de la hanche, en degrés par rapport au mouvement maximal, est plus restreinte dans au moins une direction prédéfinie que dans une quelconque autre direction, lorsque implanté.
